# EUROPEAN PATENT APPLICATION

(11) **EP 2 653 182 A1**
(43) Date of publication of application: **23.10.2013**
(21) Application number: 11849630.6
(22) Date of filing: 15.12.2011
(51) Int. Cl.: A61M 15/00

(54) **POWDER MEDICAMENT MOUTHPIECE CONTAINER AND APPLICATION**

(30) Priority: 17.12.2010 CN 201010596096
(71) Applicant: Chen, Qingtang, Yueqing, Zhejiang 325606 (CN)
(72) Inventor: CHEN, Xin, Nanjing Jiangsu 210002 (CN); CHEN, Qingtang, Yueqing, Zhejiang 325606 (CN)
(74) Representative: Altenburg, Bernardus Stephanus Franciscus
(86) International application number: PCT/CN2011/084067
(87) International publication number: WO 2012/079523

(57) **Abstract**

A powder medicament mouthpiece container comprising a container base (41) and a container cover (42). A protruding part (45) is arranged on one side of the container base (41), and a connector (46) is arranged on the protruding part (45) for connecting to a suction tube (1) of the powder medicament mouthpiece. A through hole (48) is arranged on the other side of the container base (41), and the opening of the through hole (48) is arranged in alignment with same of a cavity duct (49) of the container base (41). The powder medicament mouthpiece container can be used to hold the powder medicament mouthpiece, and can be connected to the powder medicament mouthpiece to form an inhaler. Employment of a filter within the powder medicament mouthpiece to separate powder medicament from dry powder allows for improved efficacy.

## Description

### Technical field

The present invention relates to a medical device, in particular to a medicinal powder nozzle container and its application. Specially, the container is connected with the nozzle to form an inhaler or directly connected with other dry inhaler and separate medicinal powder from the dry powder.

### Background of the invention

Inhalation treatment of asthma using present dry powder inhaler has a GINA cure rate as low as 5%. The medicinal powder particles used for inhaling is easy to congregate and difficult to separate; its dosage for each time is too little for split charging, resulting in a lactose content as high as 98.8% in the dry powder. However, long-term inhalation of lactose has some side effects, while reducing inhalation of dry powder for alleviating lactose intake will lead to reduction of dosage of the medicinal powder, thereby lead to poor treatment effect.

### Disclosure of the invention

### Technical problem

The object of the present invention is separating the medicinal powder by using the medicinal powder nozzle container and medicinal powder nozzle, and improving therapeutic effect.

### Technology solution

The object of the present invention is achieved by such way: a medicinal powder nozzle container, including a container base (41), a container cover (42); characterized in that, a projecting portion (45) is provided on one side of the base (41), a connector (46) is provided to connect with the suction tube (1); a hole (48) is provided at the bottom of the container base (41) and communicated with the channel (49).

In the present invention, at least one container wall segment (43) is provided between the base (41) and the cover (42) for adjustment of the height of the container wall.

In the present invention, inside the container wall of the base (41) or the container wall segment (43), there is at least one gasket (50), which is inserted into the gap between the container wall and placed dry powder inhaler for preventing air leakage.

In the present invention, a cap (47) is provided on the suction tube connector (46).

In the present invention, a stabilizing ring (44) is provided on the top of the cover (42) to fix the medicinal powder nozzle.

In the present invention, the connector (46) on the base (41) is connected with at least one suction tube (1) to form a medicinal powder inhaler, through which the dry powder can be poured into the hole(48) at the bottom of the container, or the Turbuhaler (36) is rotated to inhalation state at first, then is inverted and placed in the base (41), the mouth of the Turbuhaler (36) for sucking the dry powder being aligned to the hole (48) for inhaling medicine.

In the present invention, a connecting inner tube (8), sphere body (8α), and fence are provided inside the channel (49).

In the present invention, a filter part (2) is provided in the channel from the hole (48) in the container base to the sucking mouth, wherein, the mesh number, shape, size and level are set according to need, for example, a filter hole can prevent particles with a diameter of larger than 6 microns.

### Beneficial effects

The advantages of the present invention: 1. the container is connected with the nozzle in the container to form an inhaler; 2. the medicine powder particles with a diameter of less than 5 microns can be filtered out from the dry powder; 3. a relatively closed suction channel for inhaling the medicinal powder timely; 4. connection with the present dry powder inhaler to make good for deficiency; 5. for placement of the nozzle.

### Description of drawings

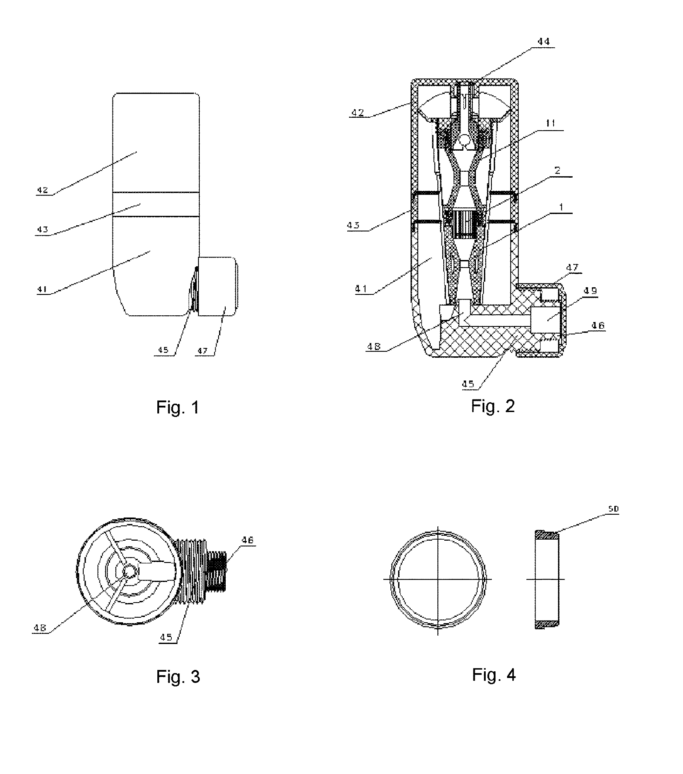

Figure 1 shows the schematic view of the appearance of the present invention.
Figure 2 shows sectional view of the present invention and the medicinal powder nozzle placed in the container.
Figure 3 shows a plan view inside the container base (41).
Figure 4 shows the schematic view of the gasket (50).
Figure 5 shows the sectional view of the base (41) connected with the nozzle.
Figure 6 shows sectional view of the present invention connected with the Turbuhaler (36).

### Embodiments of the present invention,

The present invention will be further illustrated with reference to the figures.

### Example 1:

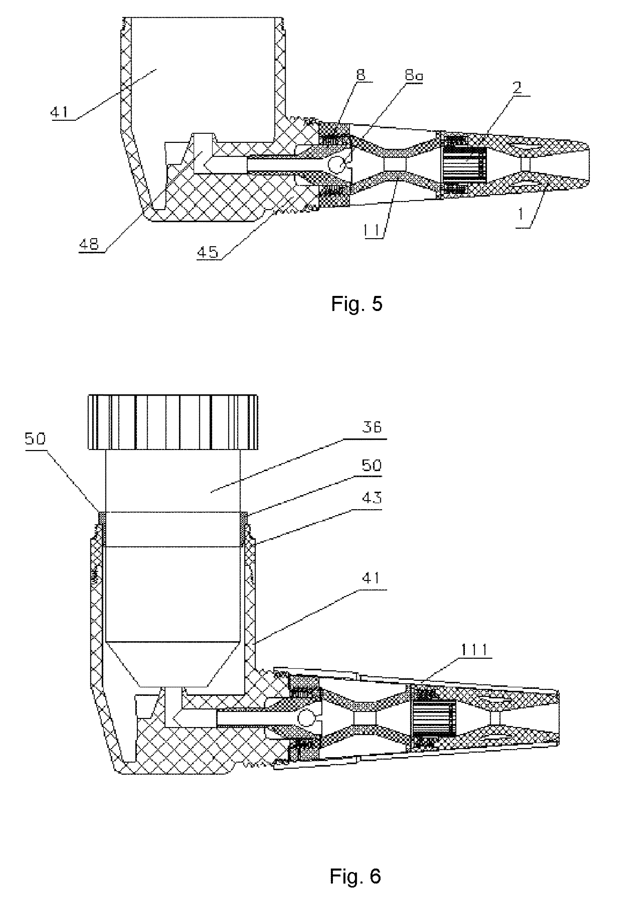

Place the filter part (2) into the suction tube (1) or in the tube cavity between the suction tube (1) and the suction tube (11), connect the suction tube (1) with the suction tube (11), remove the cap (47), place the inner tube (8) and the sphere body (8a) into the channel (49), connect the suction tube (11) with the connector (46), and finally form the powder inhaler (see Figure 5). Pour the powder for inhaling into the hole (48), when the user inhales at the suction port, the powder enters into the inner tube (8) and the medicinal powder particles are separated because of the vibration of sphere body (8a), the medicinal particles are filtered out through the filter bowl (2) and enter the respiratory tract quickly, and good effect is achieved due to sufficient amount. Inadvertent expiration into the inhaler may cause the sphere body (8a) to block narrow portion of the inner tube (8) and thereby prevent stocking medicinal powder from being forced out. After inhalation, unpick and wash the inhalers for reuse.

The device of the present invention can be made by plastic injection molding, or each part of device of the present invention is made of metal, ceramic, new materials and then connected with each other.

## Claims

1. A medicinal powder nozzle container, comprising a container base (41), a container cover (42), wherein, a projecting portion (45) is provided on one side of the base (41), a connector (46) is provided to connect with the suction tube (1); a hole (48) is provided at the bottom of the base (41), and connected with the channel (49).

2. The medicinal powder nozzle container according to claim 1, wherein, at least one container wall segment (43) is provided between the base (41) and the cover (42) for adjustment of the height of the container wall.

3. The medicinal powder nozzle container according to claim 1 or 2, wherein, inside the container wall of the base (41) or the container wall segment (43) there is at least one gasket (50), which is inserted into the gap between the container wall and dry powder inhaler to prevent air leakage.

4. The medicinal powder nozzle container according to claim 1,wherein, a cap (47) is provided on the suction tube connector (46).

5. The medicinal powder nozzle container according to claim 1, wherein, a stabilizing ring (44) is provided inside on the top of the cover (42) to fix the medicinal powder nozzle.

6. Use of the medicinal powder nozzle container according to claim 1, wherein, the connector (46) on the base (41) is connected to at least one suction tube (1) to form a medicinal powder inhaler, the dry powder for inhalation can be poured into the hole(48) at the bottom of the container, or the Turbuhaler (36) is rotated to inhalation state, then inverted and placed in the base (41), the mouth of the Turbuhaler (36) for sucking the dry powder is aligned to the hole (48) for inhaling administration.

7. Use of the medicinal powder nozzle container according to claim 1 or 6, wherein, a connecting inner tube (8), a sphere body (8α), and a fence are provided inside the channel (49).

8. Use of the medicinal powder nozzle container according to claim 1, 6 or 7, wherein, a filter part (2) is provided in the channel from the bottom hole(48) of the base to the sucking mouth, and the mesh number, shape, size and level are set according to need, for example, a filter can prevent particles with a diameter of larger than 6 microns.

## Amended claims

### Amended claims under Art. 19.1 PCT

**1.** A medicinal powder nozzle container, comprising a container base (41), a container cover (42), wherein a projecting portion (45) is provided on one side of the base (41), a connector (46) is provided to connect with a suction tube (1); a hole (48) is provided at the bottom of the base (41), and connected with the channel (49), a cap (47) is provided on the connector (46).

**2.** The medicinal powder nozzle container according to claim 1, wherein at least one container wall segment (43) is provided between the base (41) and the cover (42) for adjustment of the height of the container wall.

**3.** The medicinal powder nozzle container according to claim 1 or 2, wherein inside the container wall of the base (41) or the container wall segment (43) there is at least one gasket (50), which is inserted into the gap between the container wall and dry powder inhaler to prevent air leakage.

**4.** The medicinal powder nozzle container according to claim 1, wherein a cap (47) is provided on the suction tube connector (46).

**5.** The medicinal powder nozzle container according to claim 1, wherein a filter part (2) is placed in the suction tube (1), the connector (46) on the base (41) is connected to at least one suction tube (1) to form a medicinal powder inhaler.

**6.** The medicinal powder nozzle container according to claim 1 or 5, wherein a connecting inner tube (8), a sphere body (8α), and a fence are provided inside the channel (49).

**7.** The medicinal powder nozzle container according to claim 1, 6 or 7, wherein a filter part (2) is provided in the channel from the bottom hole (48) of the base to the sucking mouth, and the mesh number, shape, size and level are set according to need, for example, a filter can prevent particles with a diameter of larger than 6 microns.
